# EUROPEAN PATENT APPLICATION

(11) **EP 0 894 794 A1**
(43) Date of publication of application: **03.02.1999**
(21) Application number: 97830400.4
(22) Date of filing: 31.07.1997
(51) Int. Cl.: C07D 295/08, A61K 31/445

(54) **Optical isomers of cloperastine**

(71) Applicant: AESCULAPIUS FARMACEUTICI S.r.l., I-25125 Brescia (IT)
(72) Inventor: Puricelli, Laura, 25133 Brescia (IT)
(74) Representative: Moretti, Giorgio

(57) **Abstract**

The present invention regards the optical isomers of cloperastine, a compound having the following formula:

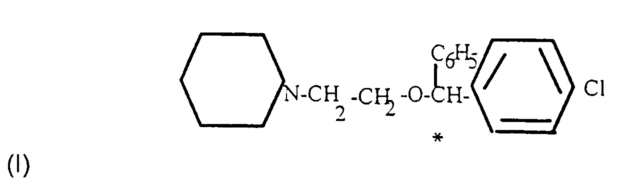

On account of the presence of the asymmetrical atom of carbon, indicated by the asterisk, the compound (I) exists in two isomeric forms, one dextrorotatory and the other levorotatory.

Described herein is the levorotatory enantiomer L(-) cloperastine and its salts (e.g., hydrochloride - fendizoate) which has made it possible to obtain a more active, less toxic product having fewer side effects compared to the D(+) isomer and the racemic mixture currently available on the market.

In the clinical field, this means a better tolerability and safety of use of the drug.

## Description

The subject of the present invention is the levorotatory form L(-) cloperastine (L-CLO) of DL-cloperastine, a drug with antitussive activity widely used in therapy.

It has in fact been surprisingly found that the biological properties of L(-) cloperastine present altogether particular and unexpected characteristics.

Studies conducted at the University of Padua have demonstrated that levorotatory L(-) cloperastine (L-CLO) is at the same time more active and less toxic than dextrorotatory D(+) cloperastin e(D-CLO) and than the racemic mixture DL.

The result of this dual difference is a considerable improvement in the therapeutic index (approximately 3 times as high) of L-CLO as compared to D-CLO.

In the case of L-CLO, the greater antitussive activity and the reduced toxicity is accompanied by an unexpected reduction in the side effects at the level of the central nervous system (CNS), on which also the therapeutic activity is exerted.

Experimental tests carried out at the University of Padua have documented these differences:
1) Comparing the antitussive activity of the L(-) enantiomer and the DL racemic mixture, the former has proved more effective (Table 1).
2) Comparing the acute toxicity (LD₅₀) of the two enantiomers, L(-) proved substantially less toxic than D(+) (Table 2).
3) From a study comparing the interaction of the enantiomers and the racemic mixture with a central stimulant, namely pentylenetetrazole (Table 3), it was confirmed that the L(-) form, at the appropriate dose level, potentiates the effects of pentylenetetrazole significantly less than the comparison substance.
4) From a study comparing the interaction of the enantiomers and the racemic mixture with a central depressant, namely hexobarbital (Table 4), it was demonstrated that the levorotatory isomer, at the dose of 5 mg/kg (a dose which is already higher than the doses normally administered in therapy) had a central depressant effect which could be detected as a potentiation of the duration of narcosis from barbiturates, which was extremely modest, for doses of 15 mg/kg there was a substantial increase in the depressant action on the CNS, and for higher doses (50 mg/kg) a reduction of depression which assumes an opposite sign (i.e., excitation) for higher doses (150 mg/kg).

The behaviour of the D(+) and DL forms proved similar, but with apparently opposite depressant effects: markedly high at 5 mg/kg, and reduced at 15 mg/kg.

The reduction in the narcotic effect at the latter dose is due to the excitatory action of the D(+) form, which counteracts the depressant effect of the hexobarbital.

The combined analysis of these results reveals for the L(-) form:
a) higher activity;
b) lower toxicity.
   These parameters result in a considerable improvement in the corresponding therapeutic index.
c) Reduction in side effects on the CNS both at therapeutic dose levels (sleepiness) and in the case of overdosage (excitation).

For therapeutic doses, depression of the CNS was found to be practically absent.

The phenomenon of major interest that it is here intended to emphasize is that the usual and expected close correlation between depression of the specific bulbar centre of coughing (antitussive effect) and depression at the level of the CNS, which is evident for the D(+) and DL forms, does not apply to the L(-) form.

At therapeutic dose levels, in the case of the L(-) form the increase in therapeutic activity is surprisingly associated with a reduction in the depression of the CNS, a secondary effect which is dominant at these dose levels.

This results in a pharmacological and toxicological profile which does not fit into the known and documented cases for the enantiomers of other substances.

The favourable pharmacological and toxicological profile has important practical implications, ensuring an effective antitussive therapy characterized by extreme tolerability and convenience, compared to treatment with the racemic mixture at present in use.

L(-) cloperastine is generally used in the form of salts with pharmacologically acceptable acids, e.g., hydrochloride - fendizoate, etc.

To provide illustrations, a number of examples are given of preparations which, however, do not exhaust the possibilities of the invention.

The process of synthesis according to the present invention is simple to carry out, with rather reduced reaction times, and gives rise to an end product that has excellent yield and moreover presents contained production costs.

In fact, even though it is a process of synthesis that involves at least 6 steps, the final yield is higher than 93%.

The economy of the process is further increased by the recovery, which can be repeated a number of times in subsequent preparations, of the principal and most costly intermediates of synthesis, such as the optically active base used in the preparation of L(-) 4-chlorobenzhydrol and the re-use after racemization of the D(+) 4-chlorobenzhydrol by-product.

These possibilities of re-use further enable a reduction in the quantities of waste liquid produced and the corresponding disposal costs.

Finally, but of no less importance, the process of synthesis of the present invention makes it possible to obtain a product of very high purity, with a D(+) enantiomer content ≤ 2%.

The compound according to the present invention may be formulated in pharmaceutical compositions mixed with a pharmaceutically acceptable excipient or diluent.

The pharmaceutical preparations may be in solid form (such as capsules, tablets, sugar-coated pills with immediate or delayed action, single-dose packets, etc.), in liquid form (solutions or suspensions ready for use or to be prepared as required); suppositories and injectable solutions.

In antitussive treatment the compound according to the invention may be administered orally or topically in dosage units containing, for example, from 2.5 mg to 70 mg of active principle, twice or more times a day, or via injection in dosage units containing, for example, from 1 mg to 20 mg of active principle, twice or more times a day.

The invention will now be described in greater detail in the following non-limiting examples.

### Example No. 1 - Preparation of L(-) cloperastine fendizoate

### Phase a) Preparation of DL 4-chlorobenzhydryl-hemisuccinate

In a 2-litre flask equipped with mechanical agitator, thermometer, and reflux refrigerant with CaCl₂ valve, put 120 g (0.55 mols) of DL 4-chlorobenzhydrol dissolved in ethyl acetate and, stirring vigorously, add 65 g (0.65 mols) of succinic anhydride, 130 ml (0.95 mols) of triethylamine, and 5 g (0.04 mols) of dimethylaminopyridine. Then heat under reflux for a few hours until the reaction is complete.

Cool to room temperature and wash the solution obtained twice using de-ionized water.

Separate the phases, and treat the organic phase with a diluted solution of hydrochloric acid and then with de-ionized water. The organic phase is dehydrated and evaporated at reduced pressure until it is dry.

The whitish residue thus obtained is purified by crystallization using toluene and n-heptane.

After filtration and vacuum-drying in an oven, 156 g of 4-chlorobenzhydryl-hemisuccinate are obtained having the following characteristics:

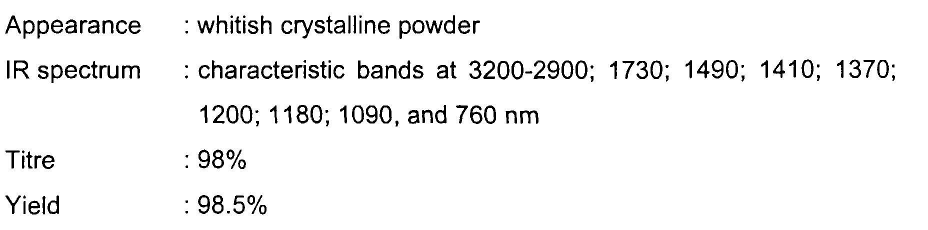

### Phase b) Resolution of optical isomers of 4-chlorobenzhydryl with an optically active base (quinine)

In a 2-litre flask equipped with mechanical agitator, thermometer, and reflux refrigerant with CaCl₂ valve, put 170 g (0.53 mols) of DL 4-chlorobenzhydryl hemisuccinate dissolved in acetone.

Heat to 45-50°C and, stirring vigorously, add, in a time range of approximately 20 minutes, a hot solution made up of 195 g (0.6 mols) of quinine dissolved in an acetone-methanol solution.

Once again under agitation, heat under reflux for 30 minutes and then cool the solution obtained to 0-5°C. Leave to rest at this temperature for a number of hours to favour complete precipitation of the product.

The whitish precipitate thus obtained, consisting of D(+) 4-chlorobenzidryl-hemisuccinate of quinine is separated by filtration and sent on to the subsequent phases of recovery and/or synthesis of D(+) cloperastine.

The solution obtained from filtration is evaporated until dry at a reduced pressure, and approximately 180 g of a whitish solid consisting of L(-) 4-chlorobenzidryl-hemisuccinate of quinine is obtained, which is sent on to the subsequent phase of hydrolysis.

### Phase c) Hydrolysis and purification of 4-chlorobenzhydrol

The solid residue obtained in the previous phase is diluted with methanol and 30% sodium hydroxide.

Heat at reflux under agitation for a number of hours and then evaporate the solvent at reduced pressure. The residue obtained is treated with water and ethyl acetate, and the phases are separated.

The organic phase is washed again with water and then treated with 2N hydrochloric acid. The aqueous phase containing the optically active base is sent on for recovery of quinine.

The ethyl acetate solution is washed to neutral pH with de-ionized water and then dehydrated and evaporated until dry at reduced pressure.

The low-melting solid thus obtained is purified by crystallization using n-heptane.

After the usual filtration and drying stages, 58 g of L(-) 4-chlorobenzhydrol are obtained having the following characteristics:

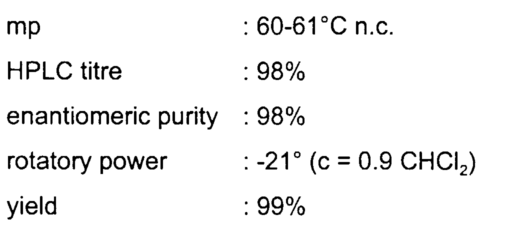

### Phase d) Preparation of L(-) cloperastine hydrochloride

In a 2-litre flask equipped with mechanical agitator, put 58 g of L(-) chlorobenzhydrol dissolved in methylene chloride and, stirring vigorously, add the chloro-ethyl-piperidine hydrochloride, tetrabutyl ammonium bisulphate and 30% sodium hydroxide.

Leave the mixture obtained under agitation for approximately 12 hours, maintaining the reaction temperature at 20-25°C.

When the reaction is complete, add de-ionized water and separate the phases.

The organic phase is washed with 35% hydrochloric acid and then with de-ionized water.

Evaporate the methylene chloride at reduced pressure to obtain a low-melting solid, which is diluted with methyl isobutyl ketone, and vacuum-dry again to remove even the last traces of methylene chloride.

The residue is diluted again with fresh methyl isobutyl ketone, and then heated until a complete solution is obtained.

Cool to 0-5°C for a few hours in order to favour complete precipitation of the product; then filter and vacuum-dry in an oven at 45-50°C.

In this way, 95 g of L(-) chloperastin hydrochloride are obtained having the following characteristics:

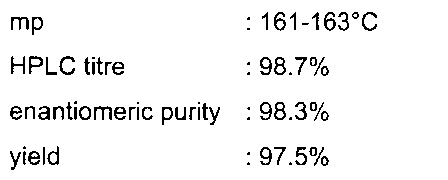

### Phase e) Preparation of L(-) cloperastine fendizoate

In a 2-litre flask put 95 g of L(-) cloperastine hydrochloride dissolved in distilled water, add ethyl acetate and, stirring vigorously, bring up to a basic pH by adding 30% sodium hydroxide, maintaining the reaction temperature at 20-25°C.

Separate the phases, and wash the organic phase with distilled water; then remove the solvent at reduced pressure.

The oily residue is dissolved in acetone and added, under vigorous stirring, to a hot solution of fendizoic acid in water and acetone.

Note the formation of a whitish precipitate, which is then cooled to room temperature and filtered and vacuum-dried in an oven at 55-60°C.

In this way, approximately 165 g of L(-) cloperastine fendizoate are obtained having the following characteristics:

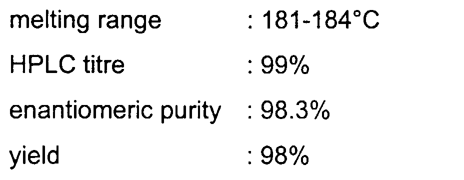

By the example described, a crystalline substance with a high degree of purity is obtained.

### Example No. 2 - Preparation of D(+) cloperastine hydrochloride

### Phase a) Hydrolysis and purification of D(+) 4-chlorobenzhydrol

the product D(+) 4-chlorobenzhydryl-hemisuccinate of quinine of Example No. 1, Phase b) is diluted with methanol and 30% sodium hydroxide. This is heated at reflux under agitation for a few hours and then the solvent is evaporated at reduced pressure. The residue obtained is treated with water and ethyl acetate, and the phases are separated.

The organic phase is washed again with water and then treated with 2N hydrochloric acid; the aqueous phase containing the optically active base is sent on for recovery of quinine.

The ethyl acetate solution is washed with de-ionized water until a neutral pH is obtained, and then dehydrated and evaporated at reduced pressure until dry.

The low-melting solid thus obtained is purified by crystallization using n-heptane.

After the usual filtration and drying stages, 58 g of D(+) 4-chlorobenzhydrol are obtained having the following characteristics:

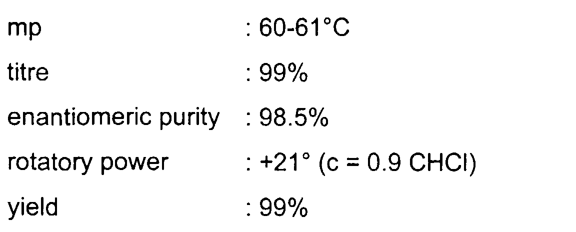

### Phase b) Preparation of D(+) cloperastine hydrochloride

In a 2-litre flask equipped with mechanical agitator, put 58 g of D(+) chlorobenzhydrol dissolved in methylene chloride and, stirring vigorously, add the chloro-ethyl-piperidine hydrochloride, tetrabutyl ammonium bisulphate and 30% sodium hydroxide.

Leave the mixture obtained under agitation for approximately 12 hours, maintaining the reaction temperature at 20-25°C.

When the reaction is complete, add distilled water and separate the phases. The organic phase is washed with a solution of hydrochloric acid and then with distilled water.

Evaporate the methylene chloride at reduced pressure to obtain a whitish solid, which is then diluted with methyl isobutyl ketone and once again vacuum-dried to remove even the last traces of methylene chloride.

The residue is diluted again with fresh methyl isobutyl ketone, and then heated until a complete solution is obtained.

Cool to 0-5°C for a few hours in order to favour complete precipitation of the product; then filter and vacuum-dry in an oven at 45-50°C.

In this way, 95 g of D(+) cloperastine hydrochloride are obtained having the following characteristics:

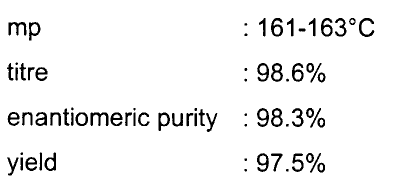

### Phase c) Preparation of D(+) cloperastine fendizoate

In a 2-litre flask put 95 g of D(+) cloperastine hydrochloride dissolved in de-ionized water, add ethyl acetate and, stirring vigorously, bring up to a basic pH by adding 30% sodium hydroxide, maintaining the reaction temperature at 20-25°C.

Separate the phases, and wash the organic phase with de-ionized water; then remove the solvent at reduced pressure.

The oily residue is melted in acetone and added, under vigorous stirring, to a hot solution of fendizoic acid in water and acetone.

Note the formation of a whitish precipitate, which is then cooled to room temperature and filtered and vacuum-dried in an oven at 55-60°C.

In this way, approximately 165 g of D(+) cloperastine fendizoate are obtained having the following characteristics:

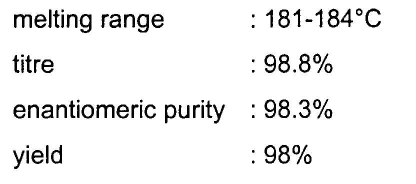

### Example No. 3 - Recovery of quinine

Pour into a reactor the washing mother liquor obtained from the preparation of L(-) and D(+) 4-chlorobenzhydrol (Example No. 1, Phase c; Example No. 2, Phase a) and possibly the D(+) 4-chlorobenzidryl-hemisuccinate of quinine obtained from the fractionated crystallization (Example No. 1, Phase b); under agitation add ethyl acetate and 20% hydrochloric acid.

Two phases are obtained, which are then separated. The aqueous phase is reextracted with fresh ethyl acetate.

From the reunited organic phases, containing D(+) 4-chlorobenzhydrol, the optical isomer is recovered.

The aqueous phase obtained above is poured into a reactor with the addition of ethyl acetate and brought up to a basic pH with 30% soda.

Separate the phases and wash the organic phase a number of times with de-ionized water, and finally with a saturated solution of sodium chloride.

Dehydrate and evaporate until dry at reduced pressure.

In this way, 175 g of basic quinine are obtained having the following characteristics:

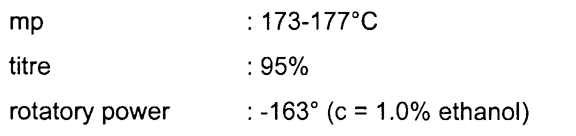

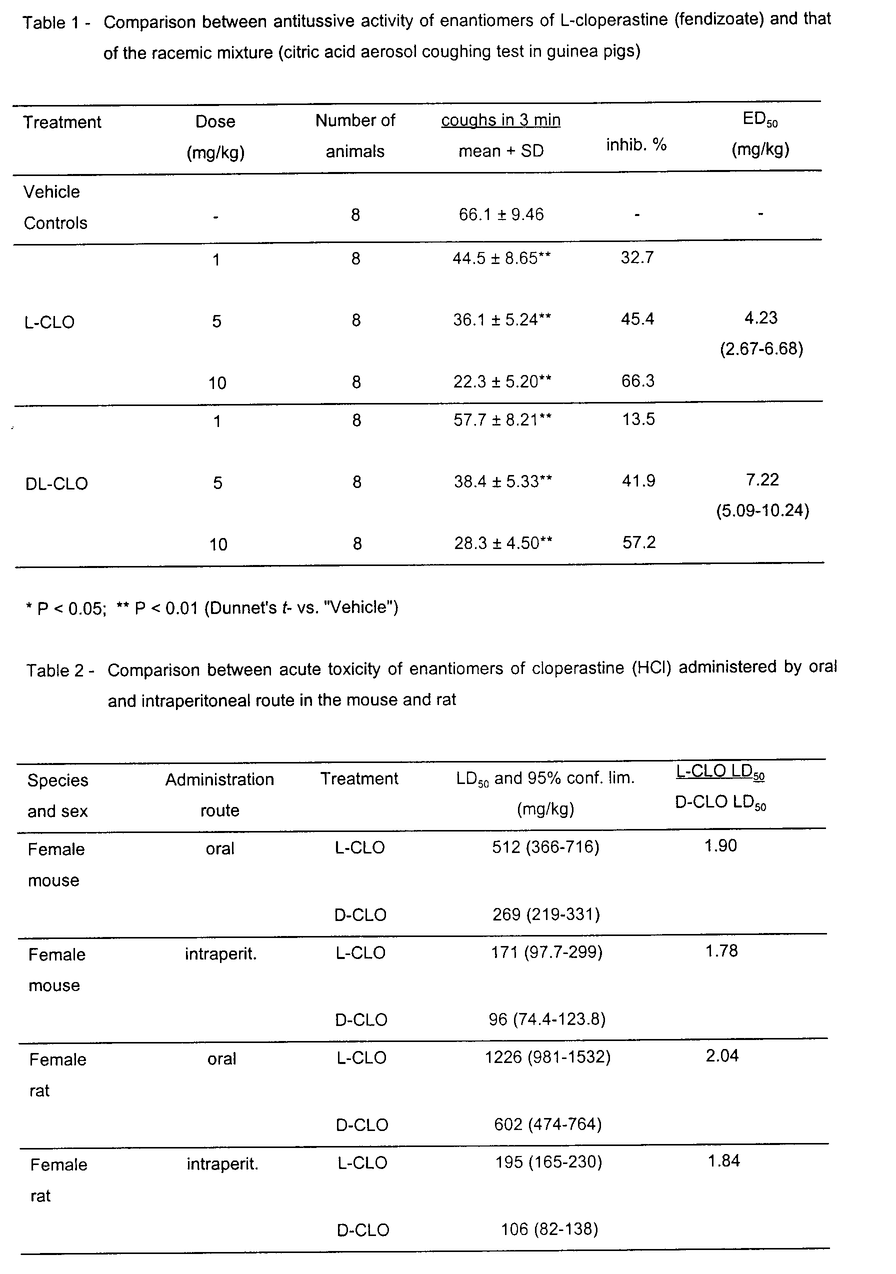

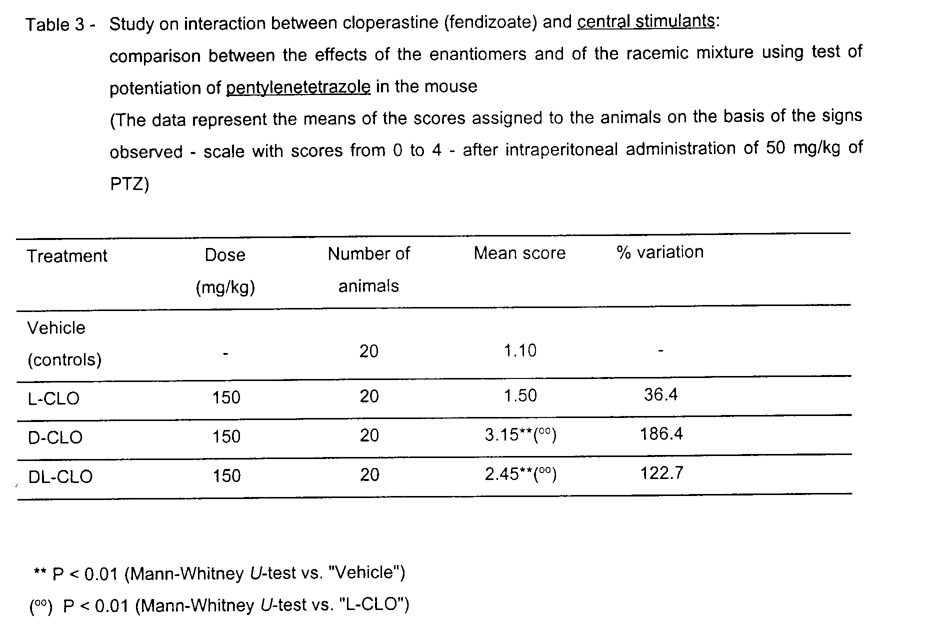

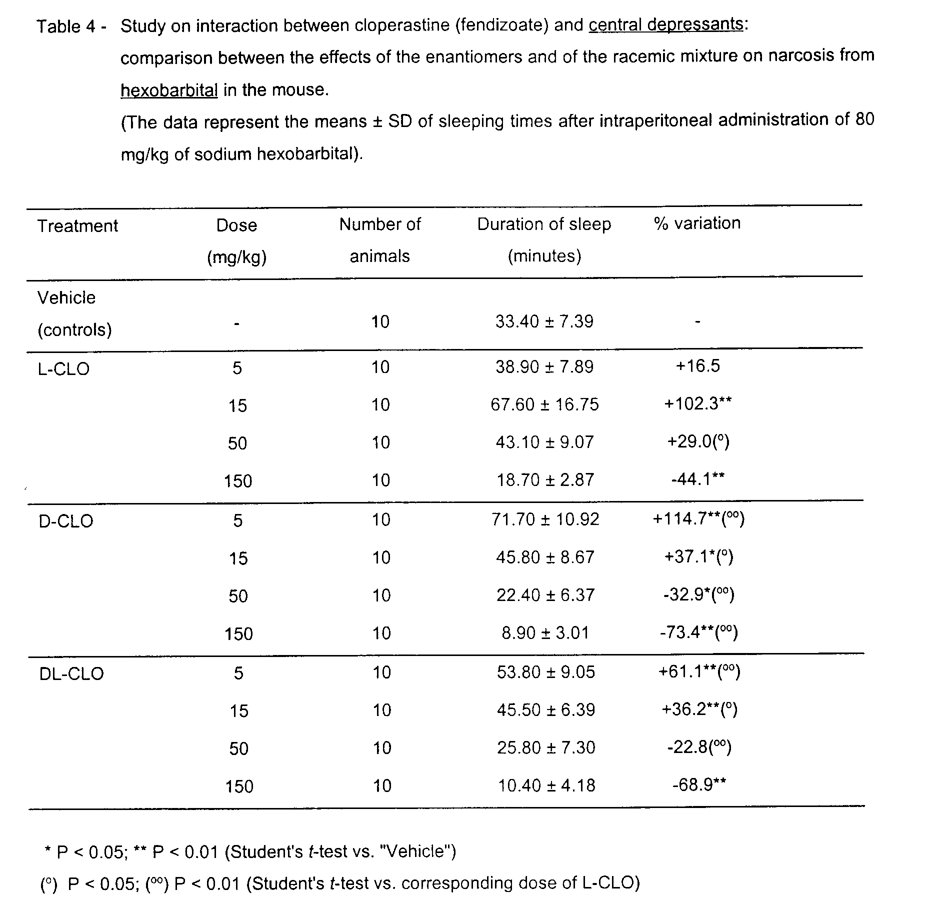

## Claims

1. Levorotatory enantiomer of cloperastine.

2. Dextrorotatory enantiomer of cloperastine.

3. Compounds of L(-) chloperastin with physiologically compatible acids.

4. Compounds of D(+) chloperastin with physiologically compatible acids.

5. Process of preparation of L(-) cloperastine fendizoate characterized by the following stages:
- resolution of optical isomers of 4-chlorobenzhydrol with optically active bases;
- preparation of L(-) cloperastine HCl;
- salification with fendizoic acid.

6. Process of preparation of D(+) cloperastin fendizoate characterized by the following stages:
- resolution of optical isomers of 4-chlorobenzhydrol with optically active bases;
- preparation of D(+) cloperastine HCl;
- salification with fendizoic acid.

7. Pharmaceutical compositions comprising a therapeutically effective quantity of L(-) or D(+) cloperastine just as it is or in the form of one of its salts either soluble or insoluble in water.

8. Pharmaceutical compositions according to Claim 7, in the form of tablets, capsules, single-dose packets, syrups, elixirs, drops, suppositories or injectable solutions.
